(19) Europäisches Patentamt
European Patent Office
Office européen des brevets

(11) Veröffentlichungsnummer: **0 000 343**
**B1**

(12) # EUROPÄISCHE PATENTSCHRIFT

(45) Veröffentlichungstag der Patentschrift: **02.09.81**

(21) Anmeldenummer: **78100240.7**

(22) Anmeldetag: **26.06.78**

(51) Int. Cl.³: **C 07 D 215/20,**
C 07 D 215/04,
C 07 D 215/36,
C 07 D 215/54,
C 07 D 219/02

(54) **Verfahren zur Herstellung von Chinolin-Derivaten.**

(30) Priorität: **02.07.77 DE 2730061**

(43) Veröffentlichungstag der Anmeldung:
**24.01.79 Patentblatt 79/2**

(45) Bekanntmachung des Hinweises auf die Patenterteilung:
**02.09.81 Patentblatt 81/35**

(84) Benannte Vertragsstaaten:
**CH DE FR GB**

(56) Entgegenhaltungen:

**Chemical Abstracts 66, 55355n (1967)**

**Chemical Abstracts 70, 87518s (1969)**

**Chemische Berichte 104, 3341—49 (1971)**

**Journal of Medicinal Chemistry 14, 17—24 (1971)**
***Seite 17, Formel 6b,e,f;**
**Seite 22, Tabel V***

(73) Patentinhaber: **BAYER AG**
**Zentralbereich Patente, Marken und Lizenzen**
**D-5090 Leverkusen 1, Bayerwerk (DE)**

(72) Erfinder: **Ehrig, Volker, Dr.**
**Odenthaler Markweg 44**
**D-5060 Bergisch-Gladbach 2 (DE)**
Erfinder: **Bien, Hans-Samuel, Dr.**
**Eichenplaetzchen 36**
**D-5093 Burscheid 1 (DE)**
Erfinder: **Klauke, Erich, Dr.**
**Eichendorffweg 8**
**D-5068 Odenthal (DE)**
Erfinder: **Schütze, Detlef-Ingo, Dr.**
**Altenberger-Dom-Strasse 170**
**D-5060 Bergisch-Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 343 B1

Verfahren zur Herstellung von Chinolin-Derivaten

Gegenstand der Erfindung ist ein neues Verfahren zur Herstellung von Chinolinen, vorzugsweise 3-Hydroxychinolinen.

Unter den zahlreichen bekannten Chinolinsynthesen ist die Methode von Friedländer, bei welcher aromatische o-Aminocarbonylverbindungen mit methylenaktiven Carbonylverbindungen alkalisch kondensiert werden, wegen ihrer universellen Anwendbarkeit eine der gebräuchlichsten.

Andererseits hat sich dieses Syntheseprinzip wegen der Schwerzugänglichkeit und der geringen Stabilität der dabei benötigten o-Amino-benzaldehyde in der Technik nicht durchsetzen können, sondern ist bisher in der Regel auf den Labormaßstab beschränkt geblieben (vgl. dazu "Heterocyclic Compounds" Bd. 4, S, 46 von R. C. Elderfield).

Es wurde nun gefunden, dass man Chinoline in vergleichsweise hohen Ausbeuten und auch im technischen Maßstab unter Anwendung des Friedländer'schen Prinzips — jedenfalls im weitesten Sinne — erhält, wenn man die verhältnismässig beständigen und gut handhabbaren aromatischen ortho-Dichloromethylisocyanate durch Hydrolyse und Decarboxylierung in die entsprechenden ortho-Aminoaldehyde überführt und diese mit $\alpha$-methylenaktiven Carbonylverbindungen kondensiert.

Das neue Verfahren eignet sich besonders zur Herstellung von Chinolinen der Formel I

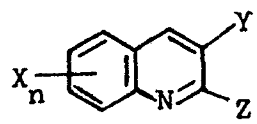

(I)

worin

X für Halogen, Nitro oder $CF_3$,

Z für Wasserstoff, Alkyl oder Aryl,

Y für Wasserstoff, OH, CN, COR oder $SO_3H$

R für Alkyl, Alkoxy oder Aryl und

n für eine ganze Zahl von 0 bis 4 stehen,

wobei die Alkyl-, Alkoxy- und Arylreste gegebenenfalls substituiert sind und Z und Y gemeinsam eine Alkylenkette oder den Rest

```
—C=CH— C=CH—
 |      |
 OH     OH
```

bilden können.

Geeignete Alkylrest weisen 1—4 C-Atome auf und können z.B. durch Halogen, CN, $C_1$—$C_4$-Alkoxy substituiert sein. Bevorzugt ist der Methylrest. Geeignete Arylreste sind Phenylreste, die durch Halogen, $NO_2$, $C_1$—$C_4$-Alkyl oder $C_1$—$C_4$-Alkoxy substituiert sein können.

Geeignete Alkylenreste weisen mindestens 3 C-Atome auf, wie z.B. $-(CH_2)-_3$ und $-(CH_2)-_4$.

Unter "Halogen" wird im Rahmen dieser Erfindung F, Br und vor allem Cl verstanden.

Falls X die Nitrogruppe bedeutet, steht n vorzugsweise für die Zahl 1.

Bevorzugt herzustellende Verbindungen entsprechen der oben genannten Formel I, worin

X für Chlor,

Z für $C_1$—$C_4$-Alkyl, vorzugsweise Methyl,

Y für OH oder —CO—$C_1$—$C_4$-Alkyl, vorzugsweise Acetyl; $COOCH_3$, $COOC_2H_5$,

n für 0 bis 4, vorzugsweise 0—2, stehen.

Bei der praktischen Durchführung des erfindungsgemässen Verfahrens geht man zweckmässigerweise so vor, dass man das Dichlormethylisocyanat in einem wäßrigen Medium, welches als Lösungsvermittler ein indifferentes organisches Lösungsmittel und ggf, ein indifferentes Tensid enthalten kann, bei Temperaturen von —5 bis 50°C, vorzugsweise 5 bis 35°C, unter starkem Rühren solange (15—90, vorzugsweise 30—45 Minuten) mit Alkali, vorzugsweise einem Erdalkalihydroxid, behandelt, bis in einer Probe kein Isocyanat mehr nachweisbar ist (z.B. IR-spektroskopisch), anschliessend — vorzugsweise nach Abkühlen auf 0—5°C und Zusatz eines Entschäumungsmittels durch Zugabe einer Mineralsäure einen pH-Wert von 4—7, vorzugsweise 5—6, einstellt und nach Beendigung der $CO_2$-entwicklung das Reaktionsgemisch in an sich bekannter Weise unter alkalischen Bedingungen mit einer $\alpha$-methylenaktiven Carbonylverbindung zu dem entsprechenden Chinolin umsetzt (Dauer 1—6, vorzugsweise 2—4 Stunden), welches nach dem Neutralstellen in üblicher Weise isoliert werden kann.

Geeignete Dichlormethylisocyanate zur Durchführung des erfindungsgemässen Verfahrens sind solche der Formel II

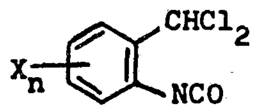

(II)

worin X und n die oben genannte Bedeutung haben.

Beispielhaft seien genannt:
2-Dichlormethyl-phenylisocyanat
3-Chlor-2-dichlormethyl-phenylisocyanat
4-Chlor-2-dichlormethyl-phenylisocyanat
4-Brom-2-dichlormethyl-phenylisocyanat
5-Chlor-2-dichlormethyl-phenylisocyanat
6-Chlor-2-dichlormethyl-phenylisocyanat
4,6-Dichlor-2-dichlormethyl-phenylisocyanat
3,4,6-Trichlor-2-dichlormethyl-phenylisocyanat
Tetrachlor-2-dichlormethyl-phenylisocyanat
5-Fluor-2-dichlormethyl-phenylisocyanat
3,4-Dichlor-2-dichlormethyl-phenylisocyanat
5-Trifluormethyl-2-dichlormethyl-phenylisocyanat
4-Nitro-2-dichlormethyl-phenylisocyanat.

Geeignete organische Lösungsmittel sind gegenüber dem Isocyanat chemisch indifferent und besitzen sowohl gegenüber dem Isocyanat als auch gegenüber der Carbonylverbindung ein gutes Lösungsvermögen.

Beispielhaft seien genannt: Benzol, Toluol, Chlorbenzol oder Xylol.

Geeignete Erdalkalihydroxide sind Magnesium-, Calcium, Strontium- und vor allem Bariumhydroxid, die in Lösung bzw. Dispersion eingesetzt werden.

Als Mineralsäure zur Einstellung des pH-Wertes von 4—7 kommt vor allem Salzsäure in Betracht.

Geeignete Carbonylverbindungen entsprechen der Formel

$$Y'—CH_2—CO—Z \quad (III)$$

worin Y' für Y oder Halogen (vorzugsweise Cl und Br) steht, wobei das Halogen im Laufe der Kondensation in OH übergeht, und Z die oben genannte Bedeutung hat.

Beispielhaft seien genannt: Acetaldehyd, Aceton, Chlor- und Bromaceton, $\omega$-Chloracetophenon, Cyclohexanon, Phloroglucin, Acetylaceton, Barbitursäure, Cyanaceton, Acetonsulfonsäure oder Acetessigester.

Die Umsetzung mit diesen Verbindungen zu den Chinolinen kann durch Zusatz üblicher Alkalien (wie NaOH, KOH, $Na_2CO_3$ oder $Ca(OH)_2$ katalysiert werden.

Die starke Durchmischung des Reaktionsgemisches, welche zur Erzielung optimaler Ausbeuten erforderlich ist, kann z.B. mit Hilfe eines Strombrechers und eines Impeller-Rührers erfolgen.

Vor der Neutralstellung des Reaktionsgemisches am Ende der Umsetzung empfiehlt es sich, ungelöstes Erdalkalihydroxid abzufiltrieren.

Das in der Endmutterlauge enthaltene Erdalkalichlorid (falls mit HCl neutralgestellt) kann durch Zusatz von Alkalihydroxid in Form des schwerer löslichen Erdalkalihydroxids wiedergewonnen werden.

Die Verbindungen der Formel I sind vielseitig anwendbar.

Beispielsweise sind Verbindungen der angegebenen Formel, worin Z für Methyl steht, Ausgangsmaterialien zum Aufbau von wertvollen Chinophthalonfarbstoffen.

Verbindungen der Formel I mit Y=OH eignen sich als Kupplungskomponenten zur Herstellung von Azofarbstoffen.

Darüber hinaus können diese Verbindungen durch Umsetzung mit Isocyanaten oder Phosphorsäureesterchloriden in die entsprechenden Carbamate bzw. Phosphorsäureester umgewandelt werden, welche insektizide Eigenschaften besitzen.

Verbindungen der Formel I mit Y=Acetyl sind z.T. wertvolle Analgetika.

### Beispiel 1

Zu einer Aufchlämmung von Bariumhydroxid [hergestellt durch Zusatz von 80 g (1 Mol) 50%iger NaOH zu einer Lösung von 125 g (0,53 Mol) Bariumchlorid] in 650 ml Wasser läßt man bei 15—18°C unter kräftigem Rühren (mit Hilfe eines Strombrechers und eines Impellerrührers) 48 g (0,24 Mol) 2-Dichlormethylphenylisocyanat zulaufen. Man rührt 30—40 Minuten bei dieser Temperatur nach und tropft bei 3—5°C ca. 100 ml 10%ige Salzsäure bis pH 5—6 in das Reaktionsgemisch, wobei $CO_2$ ent-

wickelt wird. Um ein Uberschäumen zu vermeiden, empfiehlt es sich gelegentlich ein paar Tropfen eines handelsüblichen Silikonentschäumers zuzusetzen. Nach Abklingen der $CO_2$-Entwicklung wird das Reaktionsgemisch mit ca. 120 ml 30%iger, NaOH alkalisch gestellt, wobei die Temperatur auf 8—10°C ansteigt. Nach Entfernung des äußeren Kühlbades fügt man dann 24,4 g (0,264 Mol) Chloraceton hinzu und läßt 2—4 Stunden bei Raumtemperatur nachrühren. Anschließend wird abgesaugt, der Filterrückstand mit wenig verdünnter NaOH gewaschen und das Filtrat mit ca. 130 ml 20%iger HCl auf pH 7 eingestellt. Das dabei ausfallende 3-Hydroxychinaldin wird abgesaugt, mit Wasser gewaschen und bei 70—90°C getrocknet. Ausbeute: 34,2 g (90%). Der Schmelzpunkt liegt bei 259—63°C.

Verfährt man wie vorstehend angegeben, arbeitet jedoch unter den in nachstehender Tabelle aufgeführten Bedingungen, so erhält man ebenfalls entsprechende Chinolinderivate in etwa gleich guten Ausbeuten:

TABELLE 1

| Beispiel | Isocyanat | Carbonyl-verbindung | Base | Hydrolyse-Temp. (°C) | Endprodukt | Fp. bzw. Zersp. (°C) |
|---|---|---|---|---|---|---|
| 2 | 2-Dichlormethyl-phenylisocyanat | Acetaldehyd | $Ba(OH)_2$ | 8–10 | Chinolin | Sdp. 234–36 |
| 3 | „ „ | Aceton | $Ba(OH)_2$ | 5–8 | Chinaldin | Sdp. 243–47 |
| 4 | „ „ | Chloracet-aldehyd | $Sr(OH)_2$ | 12–15 | 3-Hydroxy-chinolin | 194–97 |
| 5 | „ „ | Chloraceton | $Ca(OH)_2$ | 5–8 | 3-Hydroxy-chinaldin | 259–63 |
| 6 | „ „ | „ „ | KOH | 3–5 | „ „ | 259–63 |
| 7 | „ „ | Acetosulfon-säure | $Ba(OH)_2$ | 5–8 | Chinaldin-3-sulfon-säure | 270 |

Verfährt man wie in Beispiel 1, setzt jedoch die nachstehend aufgeführten Ausgangsmaterialien ein, so erhält man folgende Chinolinderivate.

TABELLE 2

| Beispiel | Isocyanat | Carbonyl-verbindung | Endprodukt | Fp. bzw. Zersp. (°C) |
|---|---|---|---|---|
| 8 | 3-Chlor-2-dichlormethyl-phenylisocyanat | Chloraceton | 5-Chlor-3-hydroxychin-aldin | 240 |
| 9 | 4-Chlor-2-dichlormethyl-phenylisocyanat | ,,' | 6-Chlor-3-hydroxychin-aldin | 270–75 |
| 10 | 5-Chlor-2-dichlormethyl-phenylisocyanat | ,,' | 7-Chlor-3-hydroxychin-aldin | 268–72 |
| 11 | 6-Chlor-2-dichlormethyl-phenylisocyanat | ,,' | 8-Chlor-3-hydroxychin-aldin | 203–04 |
| 12 | 5-Fluor-2-dichlormethyl-phenylisocyanat | ,,' | 7-Fluor-3-hydroxychin-aldin | 242–48 |
| 13 | 5-Trifluormethyl-2-di-chlormethyl-phenyl-iso-cyanat | ,,' | 7-Trifluormethyl-3-hydroxy-chinaldin | 250–56 |
| 14 | 3,4-Dichlor-2-dichlor-methyl-phenylisocyanat | ,,' | 5,6-Dichlor-3-hydroxy-chinaldin | 290–315 |
| 15 | 3,6-Dichlor-2-dichlor-methyl-phenylisocyanat | ,,' | 5,8-Dichloro-3-hydroxy-chinaldin | 280–95 |
| 16 | 3,4,6-Trichlor-2-dichlor-methyl-phenylisocyanat | ,,' | 5,6,8-Trichlor-3-hydroxy-chinaldin | 305–15 |

Beispiel 17

60 g (0,19 Mol) $Ba(OH)_2 \times 8H_2O$ und 14,3 g (0,042 Mol) Tetrachlor-2-dichlormethyl-phenylisocyanat werden gut vermischt und in 450 ml Wasser unter Zusatz von 1 ml Toluol eingetragen. Man läßt bei 20—25°C unter kräftigem Rühren (mit Hilfe eines Strombrechers und eines Impellerrührers) 6—8 Stunden rühren, anschließend wird unter Zugabe von 89 ml 10%iger Salzsäure bei 0—5°C ein pH-Wert von 2—3 eingestellt. Nach Beendigung der $CO_2$-Entwicklung werden 30 ml konz. NaOH zugetropft, wobei die Temperatur auf 12°C steigt. Innerhalb von 15 Minuten läßt man dann 6.5 g (0,07 Mol) Chloraceton zutropfen, wobei die Temperatur langsam auf 50°C erhöht wird. Nach 3stündigem Rühren bei 50°C wird die Reaktionsmischung bei 20—25°C mit 75 ml 20%iger Salzsäure auf pH 2 gestellt, der Rückstand abgesaugt, gewaschen und getrocknet. Umkristallisation aus o-Dichlorbenzol führt zum 5,6,7,8-Tetrachlor-3-hydroxychinaldin mit einem Zersetzungspunkt bei 270°C. Die Ausbeute liegt mit 10,5 g bei 85% d.Th.

Beispiel 18

Zu einer Aufschlämmung von 135 g (0,43 Mol) $Ba(OH)_2 \times 8H_2O$ in 500 ml Wasser läßt man bei 15—18°C unter kräftigem Rühren (mit Hilfe eines Strombrechers und eines Impellerrührers (38,4 g (0,19 Mol) 2-Dichlormethyl-phenylisocyanat zulaufen. Man rührt 30—40 Minuten bei dieser Temperatur nach und tropft bei 3—5°C ca. 95 ml 10%ige Salzsäure bis pH-5—6 in das Reaktionsgemisch, wobei $CO_2$ entsteht. Nach Abklingen der $CO_2$-Entwicklung wird das Reaktionsgemisch mit 4 ml 50%iger NaOH alkalisch gestellt. Nach Entfernung des äußeren Kühlbades läßt man dann innerhalb von 15—30 Minuten 25 g (0,25 Mol) Acetylaceton zutropfen und 4—6 Stunden bei Raumtemperatur nachrühren. Anschließend wird abgesaugt und der Filterrückstand in Methanol aufgenommen und auf 40°C erwärmt. Nach dem Abkühlen wird vom ungelösten Bariumsalz abgesaugt und das Filtrat eingedampft. Nach dem Trocknen im Exisikkator werden 33 g (94% d.Th.) 3-Acetyl-3-hydroxychinaldin vom Schmelzpunkt 54—56°C erhalten.

Verfährt man wie in Beispiel 18, setzt, jedoch die nachstehend aufgeführten Ausgangsmaterialien ein, so erhält man folgende Chinolinderivate:

TABELLE 3

| Beispiel | Isocyanat | Carbonyl-verbindung | Endprodukt | Fp. bzw. Zersp. (°C) |
|---|---|---|---|---|
| 19 | 2-Dichlormethyl-phenyl-isocyanat | Cyclohexanon | 1,2,3,4-Tetrahydro-acridin | 52 |
| 20 | ,, ,, | Acetessigsäure-äthylester | 2-Methyl-chinolin-carbon-säure-(3)-äthylester | 70–72 |
| 21 | ,, ,, | Acetessigsäure-methylester | 2-Methyl-chinolin-carbon-säure-3-methylester | 69–71 |

**Patentansprüche**

1. Verfahren zur Herstellung von Chinolinderivaten, dadurch gekennzeichnet, dass man aromatische ortho-Dichlormethylisocyanate durch Hydrolyse und Decarboxylierung in die entsprechenden ortho-Aminoaldehyde überführt und diese ohne Zwischenisolierung in an sich bekannter Weise mit $\alpha$-methylenaktiven Carbonylverbindungen umsetzt.

2. Verfahren nach Anspruch 1, dadurch gekennzeichnet, dass man als ortho-Dichlormethyl isocyanate solche der Formel II

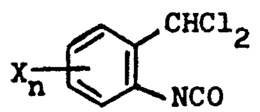

(II)

verwendet, worin
X für Halogen, Nitro oder $CF_3$ und
n für eine ganze Zahl von 0 bis 4 stehen.

3. Verfahren nach Anspruch 1, dadurch gekennzeichnet, dass man als Carbonylverbindungen solche der Formel III

$$Y'—CH_2—CO—Z \quad (III)$$

verwendet, worin
Y' für H, OH, CN, COR oder Halogen oder $SO_3H$
Z für H, Alkyl oder Aryl und
R für Alkyl, Alkoxy oder Aryl stehen, wobei die Alkyl-, Alkoxy- und Arylreste gegebenenfalls substituiert sind und Z und Y' gemeinsam eine Alkylenkette oder den Rest

```
—C=CH— C=CH—
 |      |
 OH     OH
```

bilden können.

4. Verfahren nach Anspruch 1, dadurch gekennzeichnet, dass man die Hydrolyse mittels Erdalkalihydroxiden vornimmt.

5. Verfahren nach Anspruch 4, dadurch gekennzeichnet, daß man als Erdalkali-hydroxid Bariumhydroxid verwendet.

**Revendications**

1. Procédé de production de dérivés de quinoléine, caractérisé en ce qu'on transforme des orthodichlorométhyl-isocyanates aromatiques par hydrolyse et décarboxylation en ortho-aminoaldéhydes correspondants et on fait réagir ces derniers sans isolement intermédiaire d'une manière connue avec des composés carbonyliques à groupe méthylène actif en $\alpha$.

2. Procédé suivant la revendication 1, caractérisé en ce qu'on utilise comme ortho-dichlorométhylisocyanates des composés de formule II:

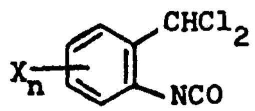

(II)

dans laquelle:

X est un halogène, un groupe nitro ou un groupe $CF_3$ et

*n* est un nombre entier ayant une valeur de 0 á 4.

3. Procédé suivant la revendication 1, caractérisé en ce qu'on utilise comme composés carbonyliques des composés de formule III:

$$Y'—CH_2—CO—Z \quad (III)$$

dans laquelle:

Y' représente H, OH, CN, COR ou un halogène ou un groupe $SO_3H$

Z représente H, un groupe alkyle ou un groupe aryle et

R est un groupe alkyle, alkoxy ou aryle, les restes alkyle, alkoxy et aryle étant éventuellement substitués et Z et Y' pouvant former ensemble une chaîne alkylénique ou le reste de formule:

—C=CH— C=CH—
   |          |
 OH       OH

4. Procédé suivant la revendication 1, caractérisé en ce qu'on conduit l'hydrolyse au moyen d'hydroxydes de métaux alcalino-terreux.

5. Procédé suivant la revendication 4, caractérisé en ce qu'on utilise, comme hydroxyde de métal alcalino-terreux, l'hydroxyde de baryum.

**Claims**

1. A process for the preparation of quinoline derivatives, characterised in that aromatic ortho-dichloromethyl isocyanates are converted by hydrolysis and decarboxylation into the corresponding ortho-aminoaldehydes and these are reacted, without intermediate isolation, in a manner which is in itself known with carbonyl compounds containing an active $\alpha$-methylene group.

2. A process according to Claim 1, characterised in that the ortho-dichloromethyl isocyanates used are those of the formula II

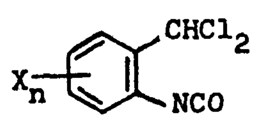

(II)

wherein

X represents halogen, nitro or $CF_3$ and

n represents an integer from 0 to 4.

3. A process according to Claim 1, characterised in that the carbonyl compounds used are those of the formula III

$$Y'—CH_2—CO—Z \quad (III)$$

wherein

Y' represents H, OH, CN, COR or halogen or $SO_3H$,

Z represents H, alkyl or aryl and

R represents alkyl, alkoxy or aryl and the alkyl, alkoxy and aryl radicals are optionally substituted and Z and Y' together can form an alkylene chain or the radical

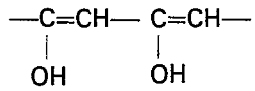

4. A process according to Claim 1, characterised in that the hydrolysis is carried out by means of alkaline earth metal hydroxides.

5. A process according to Claim 4, characterised in that the alkaline earth metal hydroxide used is barium hydroxide.